Europäisches Patentamt

European Patent Office

Office européen des brevets

(19)

(11) Publication number : **0 490 006 B1**

(12) # EUROPEAN PATENT SPECIFICATION

(45) Date of publication of patent specification :
**22.06.94 Bulletin 94/25**

(51) Int. Cl.[5] : **A61K 37/02**

(21) Application number : **90403585.4**

(22) Date of filing : **13.12.90**

(54) **Pharmaceutical compositions for the treatment of B-cell malignancies.**

(43) Date of publication of application :
**17.06.92 Bulletin 92/25**

(45) Publication of the grant of the patent :
**22.06.94 Bulletin 94/25**

(84) Designated Contracting States :
**FR**

(56) References cited :
WO-A-92/04044
BLOOD, vol. 75, no. 5, 1st March 1990, pages
1114-1118, The American Society of Hematol-
ogy, Saunders, Duluth, Mn, US; C.W. TAYLOR
et al.: "Effects of interleukin-4 on the in vitro
growth of human lymphoid and plasma cell
neoplasms"
THE JOURNAL OF EXPERIMENTAL MEDI-
CINE, vol. 168, no. 1, 1st July 1988, pages
85-94, The Rockefeller University Press, Paris,
FR; S. KARRAY et al.: "Interleukin 4 coun-
teracts the interleukin 2-induced proliferation
of monoclonal B cells"

(73) Proprietor : **SCHERING-PLOUGH**
**92, rue Baudin**
**F-92307 Levallois-Perret (FR)**

(72) Inventor : **Banchereau, Jacques**
**25 Avenue Paul Santy**
**F-69130 Ecully (FR)**
Inventor : **De France, Thierry, Charriere**
**Blanche**
**Bat. Charme,**
**Chemin de Charrière**
**F-69130 Ecully (FR)**

(74) Representative : **Beauchamps, Georges et al**
**Cabinet Z.Weinstein**
**20, avenue de Friedland**
**F-75008 Paris (FR)**

EP 0 490 006 B1

EP 0 490 006 B1

## Description

The invention relates to the use of IL-4 in the manufacture of medicaments for treating non-Hodgkin's malignant lymphomas in mammals.

Interleukin-4 [hereinafter "IL-4" but also known as B-Cell Stimulatory Factor 1, (BSF-1)] was originally described by M. Howard et al. in J. Exp. Med. (1982), Vol. 155, pp. 914-23 as a T cell-derived growth factor, distinct from IL-2, which permitted long-term tissue culture of normal mouse B lymphocytes and which interacted with activated B lymphocytes to maintain the proliferation thereof for as long as 4 months. Although mixed B lymphocyte explants have been used to initiate cultures, it appears that B lymphocytes with immature phenotype are specifically enhanced by IL-4 in tissue culture. See for example C. Peschel et al., J. Immunol. (1989), Vol. 142, 1558-1568. In addition, G. Trenn et al. J. Immunol. (1988) Vol. 140, 1101-1106 discloses that IL-4 stimulates the development of cytotoxic T cells from the Lyt-2+ subpopulation of resting murine T lymphocytes.

The mouse IL-4 gene was cloned and expressed in COS-7 cells [See T. Otsuka et al., Nuc. Acids Res. (1987), Vol. 15,333-334]. The cloned factor had all the activities in tissue culture seen for the factor purified from T cell culture supernatants. Cloning and expression of the human IL-4 gene have been described by N. Arai et a]., J. Immunol. (1989, Vol. 142, 274-282 and T. Yokota et al., Proc. Natl. Acad. Sci. (1986), Vol. 83, 5844-5848 with the factor produced in COS-7 cells having similar activities to the native molecule as studied in tissue culture. As IL-4 was studied both in human and murine cell systems, additional in-vitro activities were attributed to the molecule: (i) IL-4 played an important role in the induction and regulation of IgE synthesis, a process occuring as B lymphocyte subpopulations were induced into proliferation [See Pene, J., Proc. Natl. Acad. Sci (1988), Vol 85, 6880-6884]; (ii) IL-4 induced low affinity FcΣ receptors (CD23) on normal human B lymphocytes in tissue culture [See T. DeFrance et al., J. Exp. Med. (1987), Vol. 165, 1459-1457]; (iii) IL-4 interacted in an extremely precise way with other lymphokines, notably interferon-γ [See R.L. Coffman et al. Immunol. Res. (1988), Vol. 102, 5-27 and Pene et al., supra] and T cells [See R.L. Coffman et al. supra, Pene et al. supra, and M.D. Widmer et al., Nature, (1987), Vol. 326, 795-98] to bring about B cell proliferation and alteration; and (iv) IL-4 increased MHC class II antigen expression on resting B cells (R Noelle et al., PNAS 81. 6149-6153, 1984). T.R. Mosmann et al. in J. Immuno., Vol. 138, 1813-1816 disclosed that human and murine IL-4 which are 50% homologous at amino acid sequence 1-90 and 129-149 were species specific.

Studies in humans showed that IL-4 has an effect on monoclonal B cell tumors. S. Karray et al. in J. Exp. Med. (1988), Vol. 168, 85-94, disclose that human IL-4 suppresses the IL-2-dependent proliferation of B-type chronic lymphocytic leukemia (B-CLL) in vitro. T. DeFrance et al. J. Exp. Med. (1988), Vol 168, 1321 disclose that IL-4 inhibits the in vitro proliferation but not the differentiation of activated human cells in response to IL-2. See also D.F. Jelinek et al. J. Immunol. (1988), Vol 141; 164. C.M. Higuchi et al. in Can. Res. (1989), Vol. 49, 6487-6492, disclose that in human peripheral blood lymphocytes preactivated by IL-2, IL-4 induces lymphokine-activated killer activity (LAK). J.J. Mule et al. in J. Exp. Med. (1987), Vol. 166; 792-797, disclose that in the murine system, resting splenocytes treated with murine IL-4 alone or in combination with IL-2 generate LAK activity against fresh syngenic tumor cells in vitro. G. Forni et al. in Int. J. Can. Sup. (1989), Vol. 4; 62-65, disclose that antitumor activity can be induced by injecting murine IL-4 around the tumor draining lymph node and that when IL-4 is used in combination with a nonapeptide from human IL-1β, very active lymphokine-activated tumor inhibition (LATI) is observed. J.J. Mule et al. in J. Immuno. (1989), Vol. 142; 726-733 disclose that the major phenotype of the cells induced by murine IL-4 is surface expression of asialo-GM$_1$, Thy$^+$, Lyt$^{2+}$, T$^{3+}$ and that in LAK cells generated by a combination of IL-2 plus IL-4, there is an increase in granule-associated serine esterase. D.J. Peace et al. in J. Immuno. (1988), Vol. 140, 3679-3685 disclose that IL-4 induced LAK activity is associated with two different cell types, one NK-like (NK1.1$^+$, Lyt$^{2-}$) and the other T cells like (NK 1.1$^-$, Lyt$^{2+}$). R.I. Tepper et al. in Cell (1989), Vol. 57; 503-512 disclose that murine tumor cell lines transfected with murine IL-4 are inhibited from growing in vivo but that IL-4 transfected tumor cells mixed with nontransfected tumor cells resulted in the inhibition of the nontransfected tumor growth in vivo when the two tumor cell types were colocalized. R.I. Tepper et al. also disclose that when the nontransfected tumor was at a distal site from the IL-4-transfected tumor, inhibition was not observed. R.I. Tepper further disclose that parenteral administration of a cytokine, e.g. IL-2 or IL-4 to a tumor-bearing animal is "compromised by the short half life of the factor (as is the case for IL-2) and the need to obtain the cytokine in quantities sufficient to achieve effective dose levels. G. D'Orazi et al. in Proceedings of the American Association for Cancer Research, (March 1990), Vol. 31, p. 252, Abstract N° 1490 disclose that IL-4 induced an antitumor response in a nude mouse model.

A method of treating solid tumors in mammals afflicted with solid tumors by systemically administering IL-4 to said mammals is disclosed in WO-A-9114450.

The potential of inhibiting the growth of a variety of lymphoid neoplasms including B-Cell neoplasms is disclosed by Taylor and al. in BLOOD, vol. 75, N° 5 March 1990, pages 1114-1118. But, accordingly to these

2

document, not all kinds of B-cell lymphomas are susceptible to inhibition by IL-4.

Surprisingly, we have found that non-Hodgkin's malignant lymphomas may be treated and the growth thereof inhibited by administering an effective amount of IL-4 into mammals, such as human beings, afflicted with such B-cell malignancies.

Accordingly, the present invention has for object the use of IL-4 for the manufacture of a pharmaceutical composition for treating non-Hodgkin's malignant lymphomas in a mammal afflicted with such malignancies.

The present invention also has for object the use of IL-4 for the manufacture of a pharmaceutical composition for inhibiting the growth of non-Hodgkin's malignant lymphomas in a mammal afflicted with such malignancies.

The present invention still further has for object the use of IL-4 in the manufacture of a pharmaceutical composition for inhibiting the proliferation of non-Hodgkin's malignant lymphomas in mammals afflicted with such malignancies.

Another object of the invention is the use of IL-4 in the manufacture of a medicament for inducing an effective immune response to inhibit non-Hodgkin's malignant lymphomas growth or to effect non-Hodgkin's malignant lymphomas regression.

Still another object of the invention is the use of IL-4 in the manufacture of a pharmaceutical composition for augmenting an effective immune response to effect inhibition or regression of non-Hodgkin's malignant lymphomas in mammals afflicted with such malignancies.

The use of E-Coli derived recombinant human IL-4 in the manufacture of the above cited pharmaceutical composition is also an object of the invention.

Furthermore, a possible application of the invention is to provide a method of treating non-Hodgkin's malignant lymphomas in a mamal afflicted with such malignancies which comprises administering to said mammal an amount of IL-4 effective for such treating.

Another possible application of the invention is to provide a method of inhibiting the growth of non-Hodgkin's malignant lymphomas in a mammal afflicted with such malignancies which comprises administering to said mammals an amount of IL-4 effective for such inhibiting.

It is also a possible application of the invention to provide a method of inhibiting the proliferation of non-Hodgkin's malignant lymphomas in mammals afflicted with such malignancies which comprises administering said mammals an amount of IL-4 effective for such inhibiting.

It is still further a possible application of the invention to provide a method for inducing an effective immune response to inhibit non-Hodgkin's malignant lymphomas growth or to effect non-Hodgkin's malignant lymphomas regression which comprises administering to said mammals an amount of IL-4 effective for such inhibitings.

A method for augmenting an effective immune response to effect inhibition or regression of malignant non-hodgkin's malignant lymphomas in mammals afflicted with such malignancies is a possible application of the invention.

Figure 1 illustrates the dose-response curve of the growth-inhibitory effect in-vitro of IL-4 in accordance with this invention on the IL-2 driven proliferation of malignant B-cells from lymph nodes.

Figure 2 illustrates the dose-response curve of the growth-inhibitory effect of IL-4 in accordance with this invention on the anti-IgM-induced proliferation of, malignant B-celles from lymph nodes.

The term "B-cell malignancy" and the term "malignant B-cell" as used herein refer to non-Hodgkin malignant lymphoma (hereinafter "NHML") B-cells.

In an in vitro study, we demonstrated that IL-4 inhibited the in vitro proliferative response of freshly-isolated non-Hodgkin malignant lymphoma (hereinafter "NHML") B-cells. For this in vitro antiproliferation assay, the leukemic NHML B cells were activated with insolubilized anti-IgM antibodies or Staphylococcus Aureus Strain Cowan 1 (hereinafter "SAC"). For 8 of the 9 leukemic NHML B cells, IL-2 was the sole cytokin/B cell tropic factor which significantly and reproducibly stimulated DNA synthesis in these NHML B-cells activated through their surface Igs. IL-4 strongly suppressed the proliferative signals delivered to the NHML B cells either by anti-Ig reagents alone or by the combination of IL-2 and anti-Ig reagents. These in vitro data suggest that IL-4 essentially provides growth inhibitory signals to NHML B-cells where said B-cells are activated through their surface Ig receptors. It is expected based on these in vitro results that IL-4 would be useful in clinical treatment of mature B-cell malignancies.

The phrase "treating a B-cell malignancy" as used herein means a broad range of anti-B cell responses resulting from administration of IL-4 in accordance with this invention including: (1) effecting B-cell malignancy regression; (2) inhibiting malignant B-cell growth; (3) inhibiting the proliferation of malignant B-cells; (4) inducing an effective immune response to inhibit malignant B-cell growth or effect malignant B-cell regression; and (5) augmenting an effective immune response to effect inhibition or regression of malignant B-cell growth in mammals. The immune response of some mammals in the absence of IL-4 may not be strong enough or

3

fast enough to effect malignant B-cell growth inhibition or malignant B-cell regression but we have found effective methods which comprise administering to malignant B-cell bearing mammals an amount of IL-4, preferably recombinant IL-4, effective for each of such purposes.

Any suitable IL-4 may be employed in the present invention. Complementary DNAs (cDNAs) for IL-4 have recently been cloned and sequenced by a number of laboratories, e.g. Yokota et al., Proc. Natl. Acad. Sci. USA. (1986), Vol. 83; 5894-5898 (human); Lee et al., Proc. Natl. Acad. Sci. USA (1986), Vol. 83; 2061-2065 (mouse); Noma et al., Nature (1986), Vol. 319; 640-646 (mouse); and Genzyme Corporation, Boston, Massachusetts (human and mouse). Moreover, non-recombinant IL-4 has been purified from various culture supernatants, e.g.; Grabstein et al., J. Exp. Med. (1985), Vol. 163; 1405-1413 (mouse); and Ohara et al., J. Immunol. (1985), Vol. 135; 2518-2523 (mouse BSF-1).

Preferably, the IL-4 used in the present invention is human IL-4, and most preferably it is the human version with the sequence described in Yokota et al., Proc. Natl. Acad. Sci. USA (1986), Vol. 83; 5894-5898 and PCT Patent Application N° 87/02990 published May 21, 1987 that is expressed in and isolated from E. Coli (U.S. Patent Application N° 079,666, filed July 29, 1987 and U.S. Patent Application N° 194,799, filed July 12, 1988). The production of IL-4 from CHO cells is described in commonly-owned U.S. Patent Application SN 386,937, filed July 28, 1989. The production of IL-4 from E. coli is described in commonly-owned U.S. Patent Application SN 429,588, filed October 31, 1989.

According to this invention, mammals are administered an effective amount of an IL-4 to inhibit malignant B-cell growth, to effect malignant B-cell regression, to induce an effective immune response, to inhibit malignant B-cell growth or to effect malignant B-cell regression or to augment an effective immune response to effect solid tumor growth inhibition or solid tumor regression.

From about 0.25 to about 15 micrograms of IL-4, preferably human IL-4 ("hIL-4") recombinantly produced from E. coli or CHO cells, more preferably E. coli-derived recombinant hIL-4, per kilogram of body weight per day is preferably administered. More preferably, mammals are administered about 5 to about 15 micrograms of recombinant hIL-4 per kilogram of body weight per day, and most preferably mammals are administered about 5 to about 10 micrograms of recombinant hIL-4 per kilogram of body weight per day in single or divided doses.

The amount, frequency and period of administration will vary depending upon factors such as the level of the neutrophil and monocyte count (e.g., the severity of the monocytopenia or granulocytopenia), age of the patient, nutrition, etc. Usually, the administration of IL-4 will be daily initially and it may continue periodically during the patient's lifetime. Dosage amount and frequency may be determined during initial screenings of neutrophil count and the magnitude of the effect of IL-4 upon the increase in antibody levels.

Administration of the dose can be intravenous, parenteral, subcutaneous, intramuscular, or any other acceptable systemic method. The IL-4 can be administered in any number of conventional dosage forms. Parenteral preparations include sterile solutions or suspensions. Dosages of more than about 10 to about 15 micrograms of recombinant IL-4 per kilogram of body weight are preferably intravenously or subcutaneously (e.g. by bolus s.c.) administered to human beings.

The formulations of pharmaceutical compositions contemplated by the above dosage forms can be prepared with conventional pharmaceutically acceptable excipients and additives, using conventional techniques.

Presently, the IL-4 is preferably administered systemically via injection, preferably via subcutaneous bolus or intraperitoneal injection or even intravenous injection. The solutions to be administered may be reconstituted lyophilized powders and they may additionally contain preservatives, buffers, dispersants, etc.

Preferably, IL-4 is reconstituted with 10 millimolar citrate buffer and preservative-free sterile water with the maximum concentration not to exceed 100 micrograms per milliliter and administered systemically via subcutaneous injection, intraperitoneal injection or via continuous intravenous infusion or by intravenous injection. For continuous infusion, the daily dose can be added to 5 ml of normal saline and the solution infused by mechanical pump or by gravity.

The effect of IL-4 on B-cell malignancies can be determined inter alia by shrinkage of lymph nodes and spleen, a fall in the circulatory malignant B-cells as well as by the reduction in tumor volume (which can be measured using standard techniques such as caliper measurements, X-ray and MRI) as well as by increased life span or survival of such mammals by the following test protocol.

## MATERIALS AND METHODS

### Isolation and purification of malignant (leukemic) B cells

Pathological samples were provided by Pr. Sotto (Hospital Albert Michallon, Grenoble, France), Dr. J.F.

Rossi (Hospital Val d'Aurelle, Montpellier, France) and Dr. J.P. Magaud (Hospital Edouard Herriot, Lyon, France). Nine patients with the diagnosis of low grade non Hodgkin, non Burkitt, malignant lymphoma (NHML) B-cells according to the Kiel classification, who have not received chemotherapy for the last four months preceding surgery were selected for this study. The available specimens included 5 lymph nodes (EMZ, GAN, PP, MAI, PRO) and 4 spleens (BOU, DEL, BRE, THE). Mononuclear cells were obtained after dilaceration of the organs on a steel mesh and centrifugation of the cell suspension over Ficoll/Hypaque gradient. For B-cell purification, mononuclear cells were first submitted to E rosetting with sheep red blood cells. The non-rosetting cells (E⁻ fraction) were subsequently incubated with a cocktail of anti-T cells (anti-CD3, anti-CD2) and anti-monocytes (anti-CD14) monoclonal antibodies. Residual non-B cells were next removed from the E⁻ population after incubation with magnetic beads (Dynabeads, Dynal, Oslo, Norway) coated with anti-mouse IgG.

## Reagents

Insolubilized anti-IgM antibodies were purchased from BioRad Laboratories (Richmond, CA) and were used at the final concentration of 10 µg/mL. Formalinized particles of Staphylococcus Aureus Strain Cowan I (SAC) were purchased as Pansorbin from Calbiochem-Behring Corporation (La Jolla, CA). SAC was used at 0.005% final concentration (w/v).

## Cytokines

Purified recombinant IL-2 (3 x 10⁶ U/ml) was purchased from Amgen Biologicals (Thousand Oaks, CA) and used at the final concentration of 20 U/mL which was determined to be optimal for the growth of normal B cells co-stimulated with insolubilized anti-IgM antibodies. Purified recombinant human IL-4 (derived from E. coli, 1 x 10⁷ U/mg) was provided by Drs. P. Trotta and T.L. Nagabhushan (Schering-Plough Research, Bloomfield, NJ).

In the experiments, IL-4 was used at the final concentration of 500 U/ml which concentration provides maximal stimulation of B cell growth, as estimated on normal B cells activated with insolubilized anti-IgM antibodies.

## Cultures

The nine purified leukemic NHML B cells were cultured in Iscove's medium (Flow Laboratories, Irvine, CA) enriched with 50 µg/mL of human transferrin, (Sigma Chemical Co., St Louis, MO), 0.5% bovine serum albumin (Sigma), 5 µg/mL of bovine insulin (Sigma), 5% selected heat inactivated fetal calf serum, 100 U/ml of penicillin, 100 µg/ml of Streptomycin (all from Flow Laboratories) and 10⁻⁵M of β mercaptoethanol (Sigma). For proliferation assays, 1 x 10⁵ of leukemic B-cells were plated in 100 µL of culture medium in round-bottom microwells and incubated in a 5% CO₂ humidified atmosphere at 37°C for five days in the presence of Polyclonal B cell activators PBA-SAC, or insolubilized anti-IgM antibodies and/or factors IL-4 (500 U/ml) and/or IL-2 (20 U/mL) which were added at the onset of the culture. DNA synthesis was determined by pulsing cells with (3H)thymidine (hereinafter "(3H)TdR") for the final 16h of the culture period. Due to the heterogeneity of the responses of leukemic B cell samples, DNA synthesis was assessed at four different time intervals (on days 3, 4, 5 and 6) after onset of the culture. The results presented in Table I correspond to the time point which provided the maximal stimulation indices. Counts per minute of (3H)TdR incorporation (cpm X10⁻³) are expressed as means of triplicate determinations. Standard deviation never exceeded 10% of the mean value. The results are listed in Table I.

IL-4 was assayed for its capacity to antagonize the proliferative response of NHML B-cells to IL-2. Each of the 9 specimens of NHML B-cells was therefore co-cultured with IL-2 (20 U/ml) and IL-4 (500 U/ml) in the presence of the anti-Ig reagent previously defined as being optimal for activation. Since the leukemic samples also differed from one to another in terms of time kinetics of the response to the growth-promoting effect of IL-2, DNA synthesis was measured 4, 5, 6 and 7 days after onset of the culture. Data displayed in Table I represent, for each clone, the levels of (3H)TdR incorporation obtained at the time point corresponding to the peak of the proliferative response to IL-2. One clone (MAI) did not display significant growth response to IL-2 or IL-4 in both activation systems and therefore is not included in Table I.

In 7 out 8 cases, IL-4 failed to synergize with anti-Ig reagents to support DNA synthesis from NHML B-cells. However, one clone (BRE) exhibited the opposite pattern of response and proliferated upon culturing with IL-4 and anti-IgM antibodies. With the exception of clone BRE, all NHML B-cells samples displayed a growth response to IL-2. For all IL-2 responsive clones, IL-4 was found to significantly inhibit the IL-2 driven proliferation of the cells. The titration of the growth-inhibitory effect of IL-4 on the IL-2 induced response of

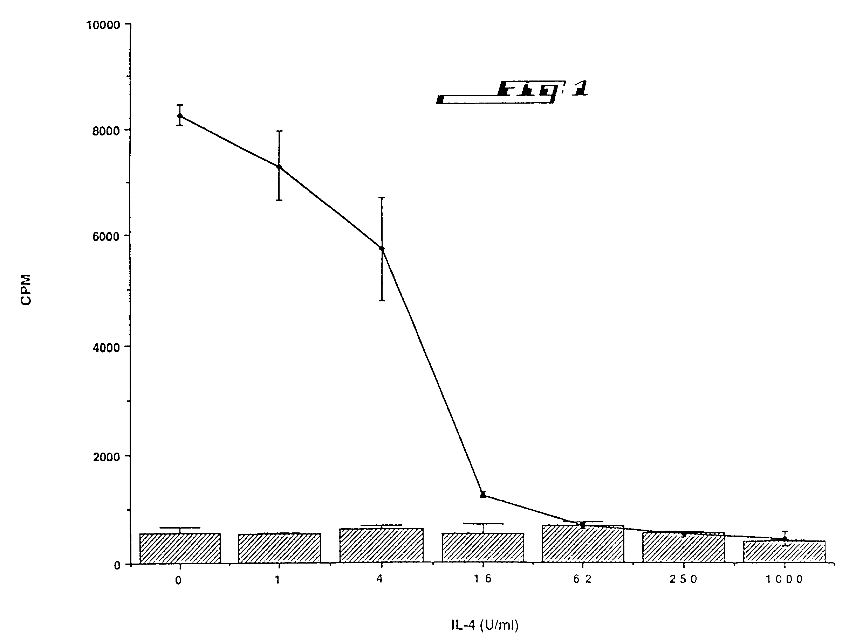

$1 \times 10^5$ cells of the leukemic clone GAN co-stimulated with 0.005% of SAC and 10 U/mL of IL-2 in the absence or in the presence of serial dilutions of IL-A. The incorporation of (3H)TdR was assessed on day 4. Figure 1 is dose-response curve which is representative of three experiments and graphically shows that complete inhibition of the response to IL-2 is already achieved for concentrations of IL-4 as low as 16 U/mL.

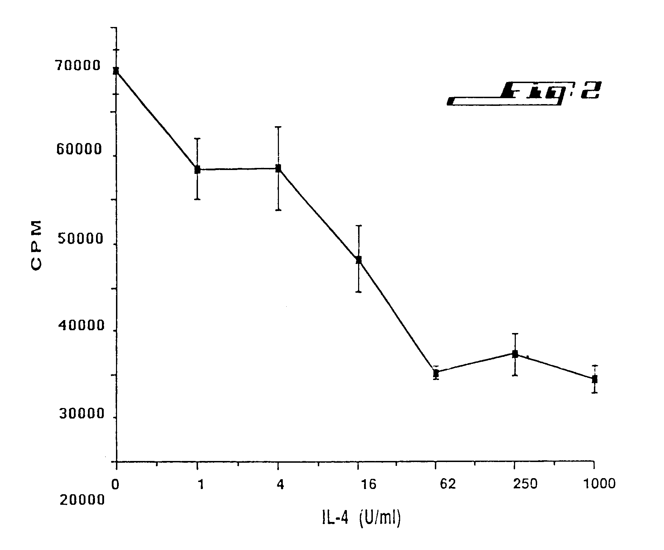

Figure 2 is a dose response curve which graphically summarizes four experiments and shows the growth inhibiting effect of IL-4 on the anti-Ig M-induced proliferation of clone PRO. These leukemic B-cells ($1 \times 10^5$ of PRO) were stimulated with insolubilized anti-Ig M antibodies (10 μg/mL), in the absence or in the presence of serial dilutions of IL-4. The (3H)TdR was added during the last 16h of a 4 day culture period. The (3H)TdR incorporation is unstimulated cultures was $346 \pm 28$ cpm. Moreover, IL-4 also inhibited the growth of the three other NHML B-cells specimens (BOU, EMZ, PRO) which already exhibited a significant proliferative response upon ligation of their surface Igs with SAC or anti-IgM antibodies (Table I, Fig. 2). In the present study, we show that, in their great majority, NHML B-cells activated through their surface Ig receptors can be stimulated for DNA synthesis by IL-2. In some tumor specimens (3 out of 9), a proliferative response could also be induced by the sole ligation of the surface Igs in the absence of exogenous growth factors. IL-4 significantly suppressed not only the growth response of these cells to IL-2 but also to the anti-Ig reagent by itself.

## TABLE I

### ANTI-PROLIFERATIVE EFFECTS OF IL-4 ON IL-2 INDUCED GROWTH OF FRESHLY ISOLATED NON HODGKIN MALIGNANT LYMPHOMA ("NHML") B CELLS[1]

| | PBA= SAC[2] | | | PBA= | | Anti-IgM | | |
|---|---|---|---|---|---|---|---|---|
| NHML B cells[3]: | BOU | THE | PP | DEL | GAN | EMZ | PRO | BRE |
| Medium | 0.5 | 0.3 | 0.2 | 1.2 | 0.6 | 0.2 | 0.3 | 0.2 |
| PBA[4] | 21.4 | 2.3 | 6.2 | 0.6 | 0.6 | 18.2 | 64.8 | 2.4 |
| PBA+IL-4[5] | 11.9 | 2.0 | 3.0 | 1.6 | 4.0 | 7.8 | 30.1 | 9.1 |
| PBA+IL-2[6] | 35.5 | 7.9 | 47.2 | 8.4 | 22.2 | 56.8 | 72.3 | 4.5 |
| PBA+IL-2+IL-4 | 12.8 | 3.9 | 9.5 | 3.7 | 9.5 | 13.6 | 27.8 | 11.1 |

1. (3H)TdR incorporation (cpm x $10^{-3}$) measured after five days and expressed as the mean of triplicate determinations.
2. SAC = Staphylococcus Aureus Strain Cowan 1.
3. $1 \times 10^5$ of NHML cells incubated at 37° for 5 days.
4. PBA = Polyclonal B cell Activator.
5. 500 U/mL of IL-4
6. 20 U/mL of IL-2

## Claims

1. Use of IL-4 for the manufacture of a medicament for treating non-Hodgkin's malignant lymphomas in a mammal afflicted with such malignancies.

2. Use of IL-4 for the manufacture of a medicament for inhibiting the growth of non-Hodgkin's malignant lymphomas in a mammal afflicted with such malignancies.

3. Use of IL-4 in the manufacture of a medicament for inhibiting the proliferation of non-Hodgkin's malignant lymphomas in a mammal afflicted with such malignancies.

4. Use of IL-4 in the manufacture of a medicament for inducing an effective immune response to inhibit the growth of non-Hodgkin's malignant lymphomas or to effect non-Hodgkin's malignant lymphomas regression.

5. Use of IL-4 in the manufacture of a medicament for augmenting an effective immune response to effect inhibition or regression of non-Hodgkin's malignant lymphomas in mammals afflicted with such malignancies.

6. The use according to one of the claims 1 to 5 wherein said medicament is in the form adapted to be intraveneously or intraperitonealy or subcutaneously administered.

7. The use according to claims 1 to 5 wherein said medicament comprises an amount of IL-4 in the range of about 0.25 to about 15 micrograms per kilogram of body weight for a daily administration.

8. The use according to one of the claims 1 to 7 wherein IL-4 is a E.Coli derived recombinant human IL-4.


## Patentansprüche

1. Verwendung von IL-4 für die Herstellung eines Arzneimittels zur Behandlung von nicht hodgkinischen bösartigen Lymphomen bei einem von solchen Bösartigkeiten heimgesuchten Säugetier.

2. Verwendung von IL-4 für die Herstellung eines Arzneimittels zur Hemmung des Wachstums von nicht hodgkinischen bösartigen Lymphomen bei einem von solchen Bösartigkeiten heimgesuchten Säugetier.

3. Verwendung von IL-4 in der Herstellung eines Arzneimittels zur Hemmung der Wucherung von nicht hodgkinischen bösartigen Lymphomen bei einem von solchen Bösartigkeiten heimgesuchten Säugetier.

4. Verwendung von IL-4 in der Herstellung eines Arzneimittels zum Hervorrufen einer wirksamen immunen Antwort zur Hemmung des Wachstums von nicht hodgkinischen bösartigen Lymphomen oder um einen Rückgang von nicht hodgkinischen bösartigen Lymphomen zu veranlassen.

5. Verwendung von IL-4 in der Herstellung eines Arzneimittels zur Steigerung einer wirksamen immunen Antwort zur Durchführung einer Hemmung oder eines Rückgangs von nicht hodgkinischen bösartigen Lymphomen bei von solchen Bösartigkeiten heimgesuchten Säugetieren.

6. Die Verwendung gemäss einem der Ansprüche 1 bis 5, bei welcher das besagte Arzneimittel in einer zur intravenösen oder intraperitonealen oder subkutanen Verabreichung angepassten Form ist.

7. Die Verwendung gemäss Ansprüchen 1 bis 5, bei welcher das besagte Arzneimittel eine sich im Bereich von ungefähr 0,25 bis ungefähr 15 Mikrogrammen je Kilogramm Körpergewicht für eine tägliche Verabreichung befindenden Menge aufweist.

8. Die Verwendung gemäss einem der Ansprüche 1 bis 7, bei welcher IL-4 eine von dem menschlichen rekombinierten IL-4 abgeleitete E. Coli ist.

**Revendications**

1. Utilisation de la IL-4 pour la fabrication d'un médicament pour traiter des lymphomes malins non hodgkinien chez un mammifère affligé de telles maladies malignes.

2. Utilisation de la IL-4 pour la fabrication d'un médicament pour inhiber la croissance de lymphomes malins non hodgkinien chez un mammifère affligé de telles maladies malignes.

3. Utilisation de la IL-4 dans la fabrication d'un médicament pour inhiber la prolifération de lymphomes malins non hodgkinien chez un mammifère affligé de telles maladies malignes.

4. Utilisation de la IL-4 dans la fabrication d'un médicament pour induire une réponse immunitaire efficace pour inhiber la croissance de lymphomes malins non hodgkinien ou pour provoquer une régression de lymphomes malins non hodgkinien.

5. Utilisation de la IL-4 dans la fabrication d'un médicament pour augmenter une réponse immunitaire efficace pour effectuer l'inhibition ou la régression de lymphomes malins non hodgkinien chez les mammifères affligés de telles maladies malignes.

6. L'utilisation conforme à l'une des revendications 1 à 5, dans laquelle ledit médicament est sous la forme adaptée à être administrée par voie intraveineuse ou intrapéritonéale ou sous-cutanée.

7. L'utilisation conforme aux revendications 1 à 5, dans laquelle ledit médicament comprend une quantité de IL-4 dans le domaine d'environ 0,25 à environ 15 microgrammes par kilogramme de poids du corps pour une administration journalière.

8. L'utilisation selon l'une des revendications 1 à 7, dans laquelle la IL-4 est un E. Coli dérivé d'une IL-4 humaine recombinante.

Fig. 1

CPM

IL-4 (U/ml)

Fig. 2